# EUROPEAN PATENT SPECIFICATION

(11) **EP 3 998 991 B1**
(45) Date of publication and mention of the grant of the patent: **26.04.2023**
(21) Application number: 20742252.8
(22) Date of filing: 17.07.2020
(51) Int. Cl.: A61F 2/24

(54) **ANNULOPLASTY DEVICE**
ANNULOPLASTIEVORRICHTUNG
DISPOSITIF D'ANNULOPLASTIE

(30) Priority: 17.07.2019 EP 19186867
(43) Date of publication of application: 25.05.2022
(73) Proprietor: HVR Cardio Oy, 02600 Espoo (FI)
(72) Inventor: KERÄNEN, Olli, 23741 Bjärred (SE); VIRTANEN, Jani, 01150 Söderkulla (FI)
(74) Representative: Invent Horizon IP
(86) International application number: PCT/EP2020/070324
(87) International publication number: WO 2021/009361

(56) References cited:
- EP-A1- 2 351 540
- US-A1- 2005 055 087
- US-A1- 2005 065 550
- US-A1- 2008 234 728
- US-A1- 2017 273 788
- US-B2- 9 149 359

## Description

### Field of the Invention

This invention pertains in general to the field of cardiac valve repair. More particularly the invention relates to an annuloplasty device, such as an annuloplasty ring or helix, for positioning at the heart valve annulus.

### Background

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure. The annuloplasty ring is typically implanted around the annulus of the heart valve.

A problem with prior art annuloplasty implants is to achieve correct positioning at the heart valve and fixate the implant in the correct position. Suturing devices for annuloplasty implants have disadvantages that makes it difficult to suture in the correct position, thereby resulting insufficient suturing strength, and also in a very time-consuming procedure, which increases the risks for the patient. Furthermore, suturing devices are often not sufficiently compact for catheter based procedures. The use of clips for positioning annuloplasty implants is also associated with challenges, in particular when implanting helix rings that are to be positioned on either side of a heart valve. Insufficient fixation of such implant lead to traumatic effects since the fixation structure must ensure the correct position of the device over time. A further problem in the prior art is thus also to achieve a reliable fixation at the annulus of the heart valve. An annuloplasty implant is intended to function for years and years, so it is critical with long term stability in this regard.

The above problems may have dire consequences for the patient and the health care system. Patient risk is increased.

EP2351540A1 discloses a delivery device having a distal hollow portion to receive an implant. The distal portion has an opening to be positioned against tissue. The delivery device has a first locking unit for locking anchoring elements in a locked spring loaded-delivery position. Anchoring elements may be moved into the tissue by withdrawing the locking unit.

US2005/0055087A1 discloses a hook delivery device having a series of hooks coupled to a housing via a pivot mandrel. Inflating a balloon drives hooks into the tissue.

Hence, an improved annuloplasty implant or device would be advantageous and in particular allowing for avoiding more of the above mentioned problems and compromises, and in particular ensuring secure fixation of the annuloplasty device, during the implantation phase, and for long-term functioning, in addition to a less complex procedure, and increased patient safety. A related method would also be advantageous.

### Summary

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect an annuloplasty device is provided comprising first and second support rings having a coiled configuration in which the first and second support rings are arranged as a coil around a central axis, wherein the first and second support rings are configured to be arranged on opposite sides of native heart valve leaflets of a heart valve, retention units, an elongate element to apply a force onto the retention units and being movable between a first state and a second state, wherein at least part of the first and second support rings comprises an interior channel configured to contain the elongate element, wherein, in the first state of the elongate element, the retention units have a retracted position, in which the retention units are arranged radially within an outer surface of the first and/or second support rings, wherein, in the second state of the elongate element, the retention units have an expanded position, in which the retention units protrude from the outer surface of the first and/or second support rings.

According to a second aspect a method of repairing a defective heart valve is provided comprising positioning first and second support rings of an annuloplasty device in a first configuration as a coil on opposite sides of native heart valve leaflets of the heart valve, and moving an elongate element between a first state and a second state to transfer retention units from a retracted position, in which the retention units are arranged radially within an outer surface of the first and/or second support rings, to an expanded position, in which the retention units protrude from the outer surface of the first and/or second support rings, wherein at least part of the first and second support rings comprises an interior channel configured to contain the elongate element.

Further examples of the invention are defined in the dependent claims, wherein features for the first aspect may be implemented for the second and subsequent aspects and vice versa.

Some examples of the disclosure provide for a facilitated positioning of an annuloplasty device at a heart valve.

Some examples of the disclosure provide for a facilitated fixation of an annuloplasty device at a heart valve.

Some examples of the disclosure provide for a less time-consuming fixation of an annuloplasty to a target site.

Some examples of the disclosure provide for securing long-term functioning and position of an annuloplasty device.

Some examples of the disclosure provide for a reduced risk of damaging the anatomy of the heart such as the annulus or the valve leaflets.

Some examples of the disclosure provide for a more secure implantation of an annuloplasty device in narrow anatomies.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1a is a schematic illustration of an annuloplasty device, in a cross-sectional view, where a retention unit has a retracted position, according to an example of the disclosure;
Fig. 1b is a schematic illustration of an annuloplasty device, in a cross-sectional view, where a retention unit has an expanded position, according to an example of the disclosure;
Fig. 2a is a schematic illustration of an annuloplasty device, in a cross-sectional view, where a retention unit has a retracted position, according to an example of the disclosure;
Fig. 2b is a schematic illustration of an annuloplasty device, in a cross-sectional view, where a retention unit has an expanded position, according to an example of the disclosure;
Fig. 3a is a schematic illustration of an annuloplasty device, in a cross-sectional view, where a retention unit has a retracted position, according to an example of the disclosure;
Fig. 3b is a schematic illustration of an annuloplasty device, in a cross-sectional view, where a retention unit has an intermediate expanded position, according to an example of the disclosure;

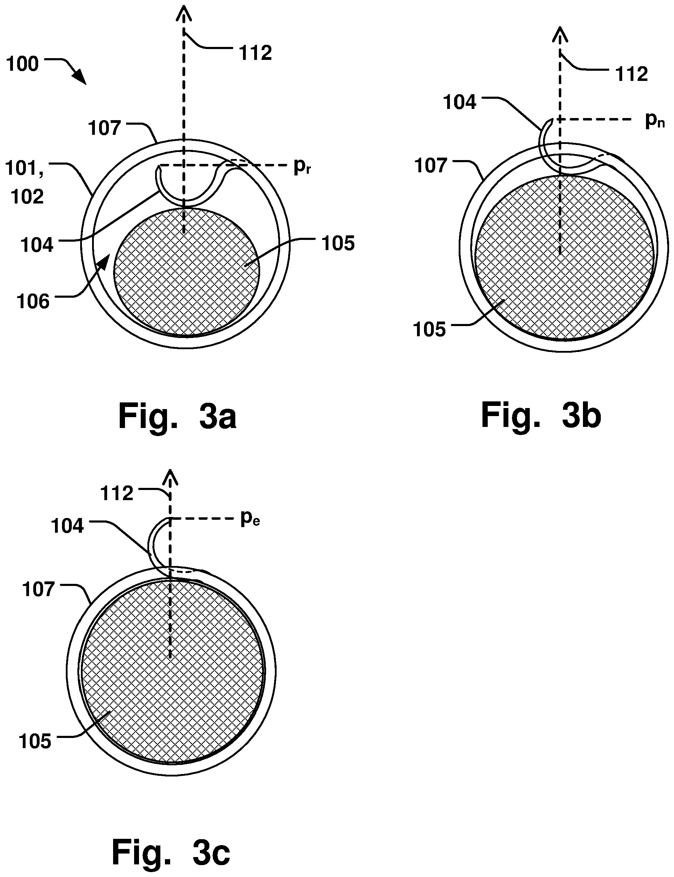

Fig. 3b is a schematic illustration of an annuloplasty device, in a cross-sectional view, where a retention unit has a further expanded position, according to an example of the disclosure;

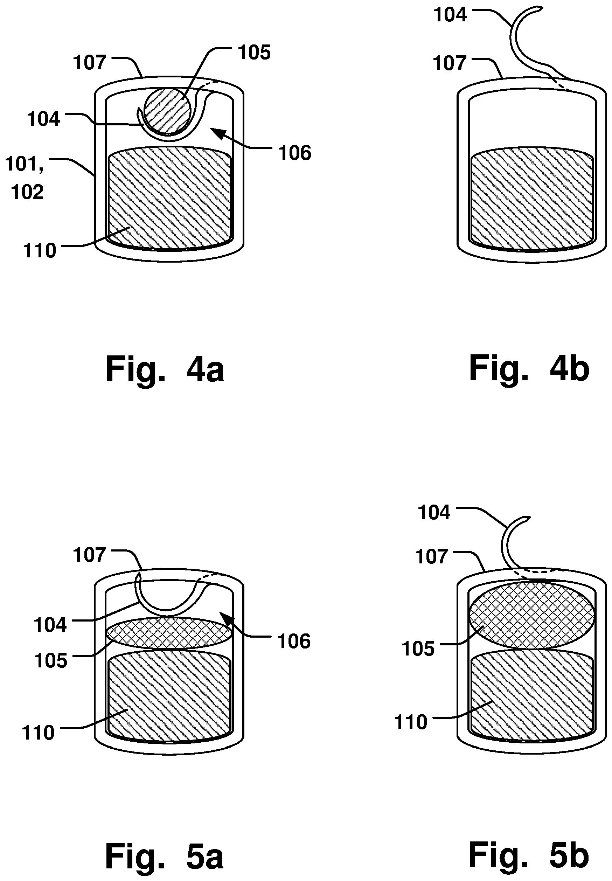

Figs. 4a-b are schematic illustrations of an annuloplasty device, in cross-sectional views, where a retention unit has retracted and expanded position, respectively, according to examples of the disclosure;
Figs. 5a-b are schematic illustrations of an annuloplasty device, in cross-sectional views, where a retention unit has retracted and expanded position, respectively, according to examples of the disclosure;

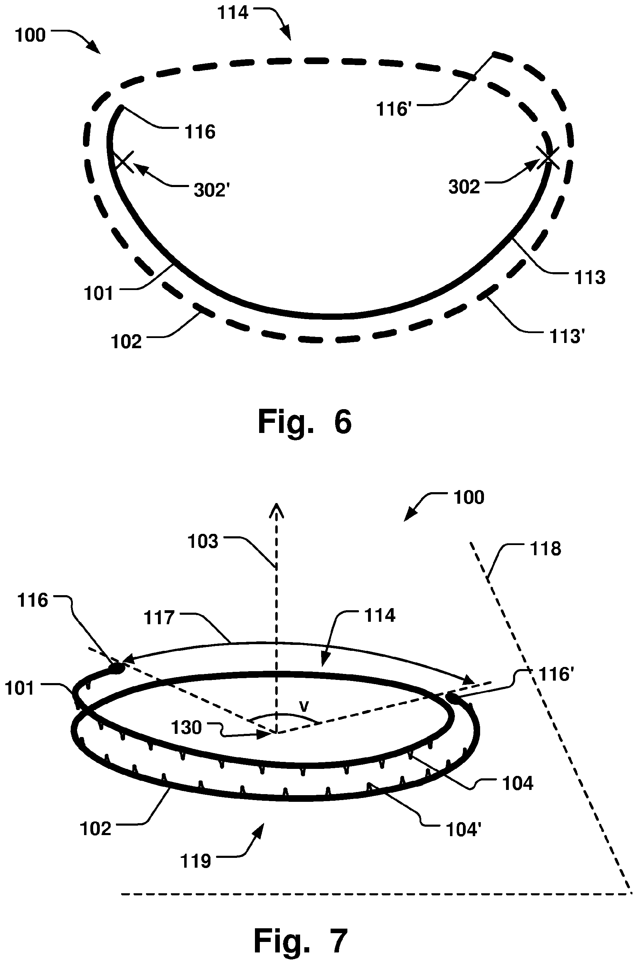

Fig. 6 is a schematic illustration of an annuloplasty device, in a top-down view, according to an example of the disclosure;
Fig. 7 is a schematic illustration of an annuloplasty device, in a perspective view, according to an example of the disclosure;

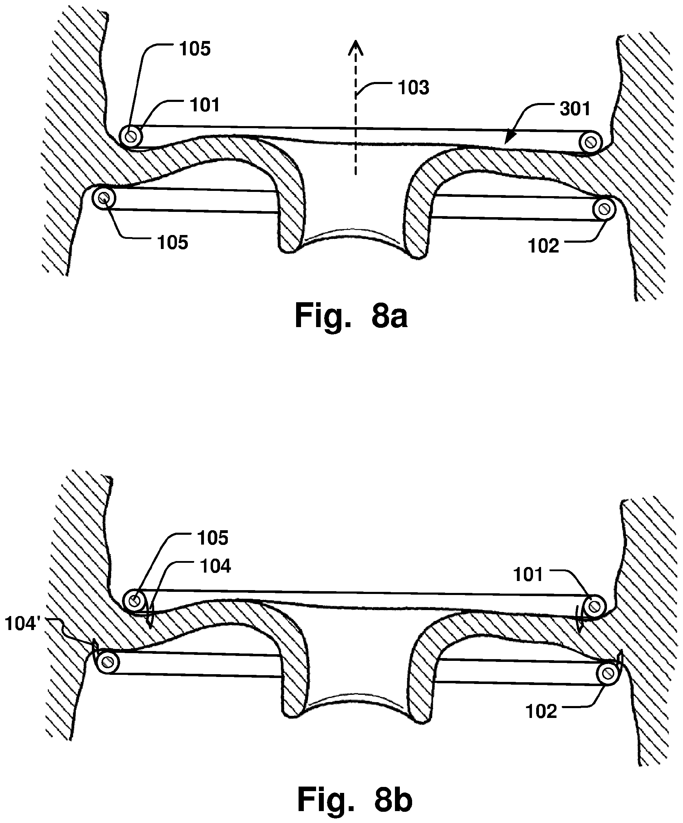

Figs. 8a-c are schematic illustrations of an annuloplasty device, in a side view, where the annuloplasty device is positioned above and below valve leaflets with retracted (8a) and expanded (8b, 8c) retention units, respectively, according examples of the disclosure;

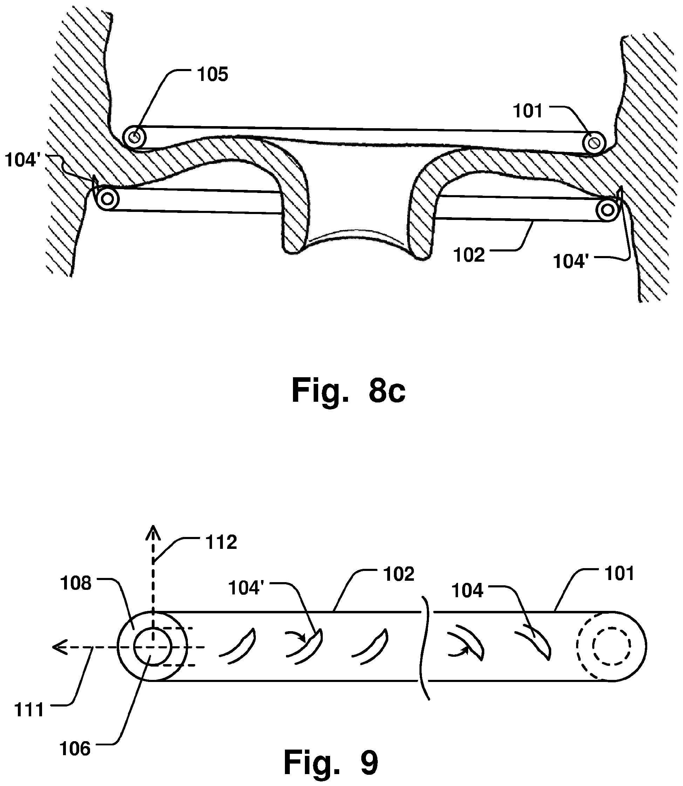

Fig. 9 is a schematic illustration of an annuloplasty device, in a side view, according to an example of the disclosure;

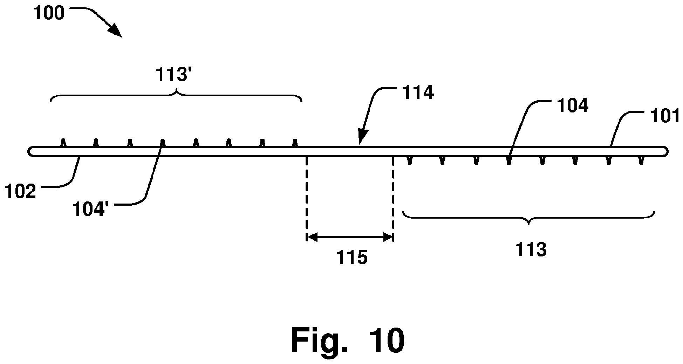

Fig. 10 is a schematic illustration of an annuloplasty device, in a side view, according to an example of the disclosure;

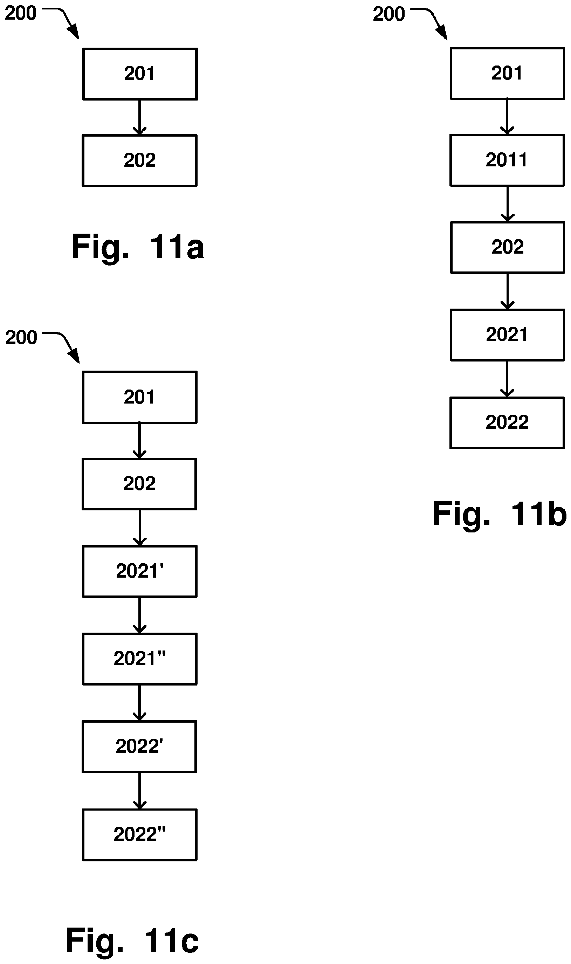

Fig. 11a is a flow chart of a method of repairing a defective heart valve, according to an example of the disclosure;
Fig. 11b is another flow chart of a method of repairing a defective heart valve, according to an example of the disclosure; and
Fig. 11c is another flow chart of a method of repairing a defective heart valve, according to an example of the disclosure.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

Fig. 7 schematically illustrates an example of an annuloplasty device 100 comprising a first support ring 101 and second support ring 102 which are adapted to be arranged as a coil, i.e. in a helix-shape, in a coiled configuration around a central axis 103, as illustrated in Fig. 7. The device 100 is arranged in the coiled configuration at least when in a relaxed state of the material from which the device 100 is formed, i.e. free from outside forces acting upon the device 100. The coil-shaped device 100 has two free ends 116, 116'. The first and second support rings 101, 102, and the respective free ends 116, 116', are configured to be arranged on opposite sides of native heart valve leaflets 301 of a heart valve, as illustrated in e.g. the side views of Figs. 8a-b. As shown in Fig. 8a, the first support ring 101 is arranged on an atrial side of the heart valve, and the second support ring 102 is arranged on a ventricular side (also shown with dashed lines in the top down view of Fig. 6, where the valve leaflets have been omitted). The second support ring 102 is illustrated with a dashed line and is in these examples arranged on the ventricular side of the heart valve, whereas the first support ring 101 is arranged on the atrial side of the heart valve. The first support ring 101 thus extend along the annulus of the heart valve on the atrial side. The first and second support rings 101, 102, are connected to form a coil- or helix shaped ring. The coil extends through the valve opening at a commissure 302 thereof, as schematically illustrated in e.g. Fig. 6. The first and second support rings 101, 102, may thus assume the coiled configuration also when in an implanted state. As explained further below, the device 100 may comprise a shape-memory material, so that the device 100 re-assumes the coiled configuration after having been delivered from a catheter (not shown) to the target site, after having been temporarily restrained in an elongated configuration of the catheter. The annuloplasty device 100, i.e. annuloplasty implant 100, may comprise a shape memory material, such as NiTiNol, or another suitable biocompatible alloy that can be heat-set in defined shapes, in a heat treatment procedure. The annuloplasty device 100 may comprise spring steel, generally of low-alloy manganese, low- or medium-carbon steel, e.g. below or around 1% carbon, which allows an annuloplasty device 100 made of spring steel to return to its original shape despite significant deflection or twisting. an alloy of medium or low carbon content, e.g. below 0.5% of carbon, such as spring steel. Alternatively, the device 100 may maintain a coiled configuration while being delivered to the target site, in which case it may be implanted at the target site for example by incision between the ribs or by opening the chest. The present disclosure, and the associated advantages described for the various examples, applies to both such variants of the device 100. The device 100 may pinch the tissue of the valve leaflets 301, between the first and second support rings 101, 102, i.e. with forces acting parallel with the central axis 103.

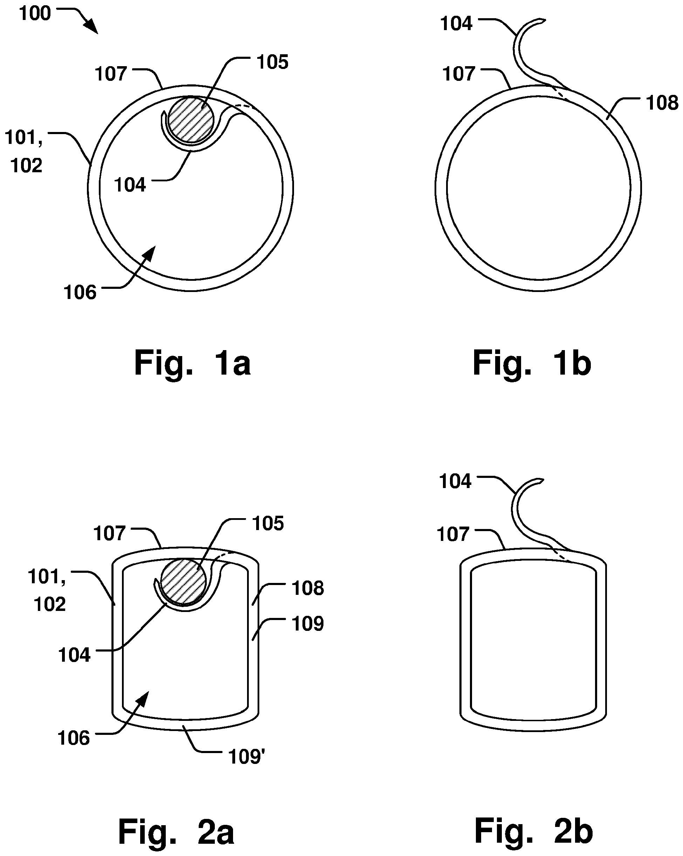

The annuloplasty device 100 further comprises retention units 104, 104', as schematically illustrated in the cross-sectional views of Figs. 1 - 5 (i.e. looking along the longitudinal direction in which the first and second rings 101, 102, extend). Although Figs. 1 - 5 only show a respective single cross-sectional profile, it is to be understood that the cross-sectional profile may correspond to any of the first and second support rings 101, 102. Figs. 9 and 10 show examples where a plurality of retention units 104, 104', are arranged on the first and second support rings 101, 102. The examples in Figs. 9 and 10 show the device 100 in an elongated stretched configuration, e.g. as arranged while being restrained in a catheter. However, as mentioned above, the device 100 assumes the coiled shape when released from the catheter, whereupon the retention units 104, 104', may engage the tissue on the atrial and ventricular sides of the heart valve, as exemplified in Fig. 8b and as described further below. The retention units 104, 104', are configured to engage the tissue of the valve and anchor the device 100 at the valve.

The annuloplasty device 100 comprises an elongate element 105 configured to apply a force onto the retention units 104, 104'. The elongate element is movable between a first state and a second state. At least part of the first and second support rings 101, 102, comprises an interior channel 106 configured to contain the elongate element 105. The elongate element 105 moves in the interior channel 106.

In the first state of the elongate element 105, the retention units 104, 104', have a retracted position, in which the retention units 104, 104', are arranged radially within an outer surface 107 of the first and/or second support rings 101, 102, as exemplified in Figs. 1a, 2a, 3a, 4a, and 5a. In the second state of the elongate element 105, the retention units 104, 104', have an expanded position, in which the retention units 104, 104', protrude from the outer surface 107 of the first and/or second support rings 101, 102, as exemplified in Figs. 1b, 2b, 3c, 4b, and 5b. Hence, the elongate element 105 in the interior channel 106 applies a force onto the retention units 104, 104', to control the position thereof. In the examples of Figs. 1a-b, 2a-b, 4a-b, the elongate element 105 applies a restraining force onto the retention units 104, 104', and the release of the restraining force allows the 104, 104', to move from the retracted position (Figs. 1a, 2a, 4a) to the expanded position (Figs. 1b, 2b, 4b). In the examples of Figs. 3a-c, 5a-b, the elongate element 105 applies a pushing force to push the retention units 104, 104', from the retracted position (Figs. 3a, 5a) to the expanded position (Figs. 3b, 5b). The retention units 104, 104', engages and pierces the tissue in the expanded position for anchoring the position of the device 100 at the heart valve. Having an elongate element 105 in an interior channel 106 to apply a force onto the retention units 104, 104', and being movable between a first state and a second state to control the position of the latter thus allows for a facilitated anchoring of the annuloplasty device 100 at the heart valve, without the need to apply sutures, clips or other external fastening devices. A precise and improved control of the position of the retention units 104, 104', is provided. Moving the elongate element 105 between the first and second states provides for an immediate actuation of the retention units 104, 104', with minimized delay. The implantation procedure may thus be accomplished in less time and with improved control. A secure positioning of the first and second support rings 101, 102, at the opposite sides of the heart valve is facilitated. At the same time, the support rings 101, 102, may be readily positioned at the correct position at the opposite sides of the heart valve before the retention units 104, 104', are deployed.

The retention units 104, 104', may be integrated with the first and/or second support rings 101, 102, as schematically illustrated in e.g. Figs. 1a-b, 2a-b, and 3a-c. By having retention units 104, 104', integrated with the first and/or second rings 101, 102, a robust, less complex and more readily implementable fixation mechanism can be provided. As illustrated in e.g. Fig. 9, a plurality of retention units 104, 104', may be provided on the respective first and second supports 101, 102. Each individual retention unit 104, 104', may engage or pierce into the tissue with a short distance, for a minimum amount of injury to the tissue. The sum of the retention force and friction created from all the retention units 104, 104', still provides for a strong fixation into the tissue. The scar healing will be quick since each individual retention unit 104, 104', as relatively small dimensions. This provides for a non-traumatic and still secure fixation of the annuloplasty device 100. Hence, the retention units 104, 104', may provide for tissue fixation at multiple points across the annuloplasty device 100 resulting in reduced forces per fixation point, and no need for bulky stitching device or knotting device. There is further no risk of coronary artery occlusion or coronary sinus perforation. Hence, the annuloplasty device 100 provides for ease of operation, and a less time consuming procedure than stitching.

The first and/or second support rings 101, 102, may be formed from a material with circumferential walls 108 enclosing the interior channel 106, as schematically illustrated in e.g. Figs. 1a-b, 2a-b, and 3a-c. The retention units 104, 104', may be formed from the material of the circumferential walls 108. This may provide for particularly robust and strong retention units 104, 104', and an overall robust fixation mechanism for the annuloplasty device 100. The retention units 104 may be formed from the material of the first support 101. Similarly, retention units 104' may be formed from the material of the second support 102. The retention units 104, 104', may be cut into shape from the material of the circumferential walls 108. The first and second supports 101, 102, may be integrated and formed from a continuous piece of material. Hence, the retention units 104, 104', may also be formed from such material.

The retention units 104, 104', may be cut to form various shapes for optimizing the gripping force into the tissue. The retention units 104, 104', may be formed by different cutting techniques such as milling or laser cutting techniques. It is also conceivable that the retention units 104, 104', are fixed or integrated onto the respective support rings 101, 102, by other methods, or by being formed from other materials.

The circumferential walls 108 may have a tubular shape enclosing the interior channel 106, as illustrated in the examples in Figs. 1a-b and 3a-c.

In other examples the circumferential walls 108 may comprise a plurality of sides 109, 109', forming a non-tubular shape enclosing the interior channel 106, as illustrated in the examples in Figs. 2a-b, 4a-b, and 5a-b. Having a non-tubular shape may allow for increasing the compression force between the first and second rings 101, 102, in the coiled configuration while maintaining a compact cross-sectional profile of the first and second rings 101, 102. The dimensions of the sides 109, 109', may be varied in order to provide for an optimized bending resistance of the support rings 101, 102. The aforementioned dimensions may also vary along the length of the first and second support rings 101, 102, i.e. along the longitudinal direction 111, so that that the flexibility of the rings 101, 102, may vary along the longitudinal direction 111 and be customized to different anatomical positions around the annulus of the heart valve. This provides for better accommodating movement of the tissue which may be a greater at localized sections of the annulus, while other sections may have an increased rigidity for a stronger pinching effect between the first and second support rings 101, 102. A more secure and robust positioning of the device 100 may thus be provided and improved long-term functioning.

The non-tubular shape may be essentially rectangular, as shown in the example of Figs. 2a-b. This may provide for particularly advantageous mechanical characteristics for increasing the compression force between the first and second support rings 101, 102, while maintaining a compact cross-section. The sides 109, 109', may be of essentially equal lengths, or different as shown in the example in Fig. 2a, and it is conceivable that the length of the sides 109, 109', are varied for optimization to different applications, and also varied along the length of the support rings 101, 102, as elucidated above.

The annuloplasty device 100 may comprise a core 110 extending along at least part of the first and/or second support rings 101, 102, as schematically illustrated in Figs. 4a-b, 5a-b. The core 110 may be formed of a material which has different mechanical properties than the material from which the first and second rings 101, 102, are formed. For example, the core 110 may be comprise a material which is more rigid than the material from which the first and second rings 101, 102, are formed. This provides for increasing and optimizing the compression force between the first and second rings 101, 102, to provide a stronger pinching of the leaflets without having to increase the cross-sectional footprint of the support rings 101, 102. It is also conceivable that the core 110 is formed from the same material as the support rings 101, 102. This may also provide for increasing the rigidity of the support rings 101, 102, since the amount of solid material through the cross-section is increased. The core 110 may extend along part of the first and second support rings 101, 102. The core 110 may also extend along essentially the entire length of the first and second support rings 101, 102. Having a core 110 provides for maintaining or increasing the compression force between the first and second rings 101, 102, while allowing for a compact cross-sectional profile and an interior channel 106 to accommodate the retention units 104, 104', in their retracted positions.

The circumferential walls 108 may enclose the core 110, as schematically illustrated in Figs. 4a-b and Figs. 5a-b. The core 110 may be a separate element which is arranged in the interior channel 106, e.g. inserted into the latter during manufacturing or during delivery or implantation of the device 100. Although being a separate element in such case it is conceivable that the core 110 may be attached to the circumferential walls 108, e.g. by welding, or an adhesive or other fixation mechanisms. In other examples the core 110 is formed as an integral piece with the walls 108 of the interior channel 106. The cross-section of the first and/or second support rings 101, 102, may in such case be solid integral piece of material having an interior channel 106 arranged in a section thereof, extending along the longitudinal direction 111. The interior channel 106 may in such case be arranged so that a thin wall with an outer surface 107 is formed e.g. in an upper part of the cross-section, as illustrated in Fig. 4a (although this example illustrates the core 110 as a separate element).

The elongate element 105 may be movable between the first and second state by being movable along a longitudinal direction 111 of the interior channel 106. This allows for a facilitated actuation of the elongate element 105, e.g. by pulling the elongate element 105, which may comprise a wire extending along the longitudinal direction 111, towards the operator after having deployed the first and second support rings 101, 102, at opposite sides of the valve leaflets.

The retention units 104, 104', may thus be restrained by the elongate element 105 to assume the retracted position in the first state, as shown in the examples of Figs. 1a, 2a, and 4a. The elongate element 105 provides for keeping the retention units 104, 104', in the retracted position while delivering the first and second rings 101, 102, to the correct positions on opposite sides of the valve leaflets. Upon moving the elongate element 105 along the longitudinal direction 111, e.g. by pulling the latter towards the operator, the retention units 104, 104', may be released to assume the expanded position, as seen in the examples of Figs. 1b, 2b, and 4b. An effective and robust deployment mechanism for the retention units 104, 104', is thus provided. The retention units 104, 104', may be deployed in the expanded position with immediate effect as soon as the elongate element 105 is pulled to release the retention units 104, 104'. The relaxed position of the retention units 104, 104', may in this example thus be the expanded position. The retention units 104, 104', thus strive to assume the expanded position when the restraining force applied by the elongate element 105 is removed. The elongate element 105 may hence be completely removed from the first and second support rings 101, 102, and removed from the implantation site and the body. Deploying the retention units 104, 104', as described by removing the elongate element 105 is particularly advantageous in that a minimum of components are left in the body after implantation of the device 100 is completed. The elongate element 105 may be pulled and removed in a stepwise manner from the interior channel 106 for selective deployment of a desired number of retention units 104, 104', into the tissue. This provides for continuously making sure that the first and second rings 101, 102, are fixed in the correct position. This provides for a particularly controlled and secure implantation procedure. Fig. 8c shows an example where the elongate element 105 has been removed from the second ring 102 and thus deploying the retention units 104' of the second ring 102, while the elongate element 105 is still arranged in the first ring 101 to restrain the retention units 104 of the first ring 101 from deployment. Fig. 8b show an example where retention units 104, 104', have been deployed from both the first and second rings 101, 102, either by complete removal of the elongate element 105 or by radial expansion of the latter as described below.

The elongate element 105 may be movable between the first and second state by being expandable in a radial direction 112 perpendicular to a longitudinal direction 111 of the interior channel 106, as schematically illustrated in Figs. 3a-c, and 5a-b. Expansion in the radial direction 112 may be provided by inflating the elongate element 105 with a fluid or gas. The elongate element 105 may thus comprise an inflatable element such as a balloon. In other examples the cross-section of the elongate element 105 may be increased in size and expanded by pushing opposite ends of the elongate element 105 towards eachother to reduce the length thereof in the longitudinal direction 111. As the length of the elongate element 105 is reduced in the longitudinal direction 111, the material from which the latter is formed is instead forced in the radial direction 112 according to the principle of conservation of the total volume of the elongate element 105. The elongate element 105 may in this case comprise a resilient material, such as a resilient polymer, e.g. a foam, rubber, or a springy wire, which is configured to expand in the radial direction 112 in response to being shortened in the longitudinal direction 111.

Thus, upon expanding the elongate element 105 in the radial direction 112, the retention units 104, 104', may be pushed by the elongate element 105 to transfer from the retracted position as exemplified in Figs. 3a and 5a, to the expanded position as exemplified in Figs. 3c and 5b. This provides for a quick and robust deployment of the retention units 104, 104'. The relaxed position of the retention units 104, 104', may in this example thus be the retracted position. The retention units 104, 104', thus strive to assume the retracted position when the pushing force applied by the elongate element 105 is removed. As elucidated further below, this provide for an improved control of the position of the retention units 104, 104', which may be gradually expanded to a desired position and also retracted if required.

Figs. 3a-c show an example where the elongate element 105 is gradually expandable to position the retention units 104, 104', at intermediate positions (pₙ) between the retracted position (pᵣ) to the expanded position (pₑ). Various intermediate positions (pₙ) may be achieved by controlling the radial dimension of the elongate element 105 to customize the penetration depth of the retention units 104, 104', for optimization to anatomical various.

In one example the retention units 104, 104', may comprise a shape-memory material. Such shape memory material may be the same material from which the first and/or second support rings 101, 102, are formed, as discussed above. Activation of the shape-memory material, e.g. by a temperature variation may cause the retention units 104, 104', to transfer from the retracted position to the expanded position. This may provide for facilitated positioning of the first and second support rings 101, 102, while the retention units 104, 104', are retracted, while an efficient fixation is attained in the implanted state of the rings 101, 102, when the retention units 104, 104', are expanded.

The first support ring 101 may be adapted to be arranged on an atrial side of the heart valve, and the second support ring 102 may be adapted to be arranged on a ventricular side of the heart valve, as exemplified in Figs. 8a-c. Fig. 6 show a schematic top-down view where the second ring 102 is shown with dashed lines and the first ring 101 is shown with a solid line. The transition point between the first and second rings 101, 102, is at the commissure 302. The first support ring 101 may comprise a first posterior bow 113 and the second support ring comprises a second posterior bow 113'. The first and second posterior bows 113, 113', may be adapted to conform to a posterior aspect of the heart valve. The first and second posterior bows 113, 113', may be separated by an intermediate anterior portion 114. The anterior portion 114 may comprise a smooth surface. I.e. the smooth surface is free from retention units 104, 104'. This is further illustrated in Fig. 10, showing the rings 101, 102, in a stretched configuration. Having a smooth surface at the anterior portion 114 reduces the risk of complications from damaging the tissue at this sensitive region of the valve. The retention units 104, 104', may thus be arranged on respective first and second posterior bows 113, 113', as illustrated in Figs. 7 and 10. This provides for avoiding piercing the tissue at an anterior portion 114, which can be associated with a greater risk of complications.

The first support ring 101 may thus comprise first retention units 104, and the second support ring 102 may comprise second retention units 104'. The first and second retention units 104, 104', may extend from the respective first and second support rings 101, 102, to produce a retention force, in use, at both of the opposite sides of the heart valve. Fig. 8b show an example where the retention units 104, 104', engage into valve tissue from the opposite sides of the heart valve. Having retention units 104, 104', at both sides of the valve provides for increasing the retention force and the strength by which the annuloplasty device 100 is fixated at the valve. The retention units 104, 104', engage the tissue from both of the mentioned sides, creating a strong retention force in the radial direction, i.e. perpendicular to the axial direction 103. The first and second supports 101, 102, pinch the tissue from both sides of the valve, so that the retention units 104, 104', a forced into the tissue. The retention units 104, 104', provides for shaping the annulus as desired even with a reduced pinching force, since the retention units 104, 104', provides for fixating the shape of the annulus in the radial direction because of the mentioned retention force. This provides for a more reliable implantation at the heart valve, both in the short term and in the long term.

The first and second retention units 104, 104', may extend in opposite directions along the axial direction 103, as illustrated in the example in e.g. Fig. 8b. I.e. the first and second retention units 104, 104', may extend from respective rings 101, 102, towards eachother, to clamp the tissue therebetween. It is conceivable however that the retention units 104, 104', may extend in different directions. The second retention units 104' may for example extend with an angle in a radially outward direction to engage tissue in a direction towards a tissue wall radially outside the annulus. Fig. 8b show only a few retention units 104, 104', for a more clear illustration, but it should be understood that a plurality of retention units 104, 104', may extend at a defined interval along the first and second support 101, 102, as shown in Fig. 7 when the device 100 has a coiled configuration.

Further, the position of the first retention units 104 may be off-set in the radial direction (perpendicular to the axial direction 103) with respect to the second retention units 104'. Thus, although both the first and second retention units 104, 104', may extend in the vertical direction, the risk of having the first retention units 104 to engage with the second retention units 104' is avoided, which otherwise may lead to fully penetrating the valve tissue. This may be realized by having different diameters of the support rings 101, 102, and/or by arranging the first and second retention units 104, 104', to extend from opposite sides (in the radial direction of Fig. 8b) of the respective support rings 101, 102.

It should be understood that in one example only the first or second support ring 101, 102, may comprise retention units 104, 104'.

The first and second retention units 104, 104', may be arranged with an off-set distance 115 from the anterior portion 114 towards respective first and second posterior bows 113, 113'. Thus, the anterior portion 114 may comprise a smooth surface free from retention units 104, 104'. I.e. the first and second retention units 104, 104', may be arranged with an off-set distance 115 from the anterior portion 114 towards respective first and second posterior bows 113, 113'. The off-set distance 114 may be varied to optimize the annuloplasty device 100 to the particular anatomy while ensuring that there is no risk of piercing the tissue at the anterior side of the valve. The first support 101 may have the retention units 104 extending in a first direction, and the second support 102 may have the retention units 104' extending in an opposite direction.

The first and second support rings 101, 102, may have respective free ends 116, 116', as illustrated in Fig. 6 and 7. The free ends 116, 116', may be configured to be arranged on opposite sides of the native heart valve leaflets. The two free ends 116, 116', may be displaced from each other with a peripheral off-set distance 117 extending in a coil plane 118, as schematically illustrated in Fig. 7. The coil plane 118 is substantially parallel to an annular periphery 119 of the coil formed by the first and second support rings 101, 102, and perpendicular to the axial direction 103. The coil plane 118 accordingly corresponds to the plane spanned by the annular periphery 119 of the device 100 when in the coiled configuration. The peripheral off-set distance 117 between the two free ends 116, 116', thus extends substantially perpendicular to the central axis 103. This means that, when the device 100 is positioned in the implanted state, around the annulus of the heart valve, the two free ends 116, 116', will be separated along the plane of the valve. By having such off-set 117 in the plane of the valve, the resulting reduced length of the first or second support rings 101, 102, will allow for reducing the number of retention units 104, 104', required to securely fixate the device 100 at the valve, while at the same time providing for a sufficient overlap of the first and second support rings 101, 102, on the opposites sides of the valve to attain a sufficiently strong pinching effect therebetween to fixate the annulus in a modified shape. In situations, placing retention units 104, 104', on the anterior side may be associated with high risk. This can therefore be avoided, by having the off-set 117 as specified. Further, the anterior portion 114 may not be provided by retention units 104, 104', as has described above.

Furthermore, the interference of the device 100 with the movements of the valve will be minimized when having an off-set 117. Fastening of the device 100 on the atrial side can thus be accomplished by fixation of the posterior bow 113, and there will be no interference on the atrial side with the movement of the valve, due to the off-set distance 117 reducing the circle sector of the first support 101. The coil of the first and second support rings 101, 102, may have a geometrical center point 130. The angle (v) between lines extending from respective free end 116, 116', and intersecting the center point 130, as illustrated in Fig. 7, may be approximately 90 degrees. The angle (v) may be in a range 80 - 120 degrees to provide for the advantageous effects as described above. The first support ring 101 may be positioned on the atrial side. The free end 116, which may connect to a delivery wire, may be arranged adjacent the anterior portion 114 or between the anterior portion 114 and the first commissure 302'. The first support ring 101 then extend past the first commissure 302' and follows the curvature of the annulus until extending through the second commissure 302, and continues to follow the valve on the ventricular side (dashed lines) as a second support ring 102. The second free end 116' may be arranged so that the second support ring 102 follows part of the shape of the anterior portion 114 of the first support ring 101. The second free end 116' may thus extend past the second commissure 302'. The two free ends 116, 116', may thus be separated with the off-set 117 along the anterior portion 114 of the device 100. In the example of Fig. 6 the off-set distance 117 is less than the length of the anterior portion 114, since the first and second support 101, 102, are curved towards eachother at the first and second free ends 116, 116'. This may provide for an improved fixation of the device 100 in some situations. However in some examples the off-set distance 117 may be increased to correspond substantially to the width of the implant along the anterior portion 114. The length of the off-set distance 117 may be between 50-100% of the length of the anterior portion 114 of the device 100. The full length of the anterior side 114 may correspond substantially to the portion of the device 100 that assumes a substantially straight extension, compared to the posterior bows 113, 113', or at least to the portion of the device 100 that extends between the anterior and the posterior commissures 302, 302'.

A method 200 of repairing a defective heart valve is disclosed. The method 200 is schematically illustrated in Fig. 11a, in conjunction with Figs. 1 - 10. The order in which the steps are described should not be construed as limiting, and it is conceivable that the order of the steps may be varied depending on the particular procedure. The method 200 comprises positioning 201 first and second support rings 101, 102 of an annuloplasty device 100 in a first configuration as a coil on opposite sides of native heart valve leaflets of the heart valve, and moving 202 an elongate element 105 between a first state and a second state to transfer retention units 104, 104' from a retracted position, in which the retention units 104, 104', are arranged radially within an outer surface 107 of the first and/or second support rings 101, 102, to an expanded position, in which the retention units 104, 104', protrude from the outer surface 107 of the first and/or second support rings 101, 102. At least part of the first and second support rings 101, 102, comprises an interior channel 106 configured to contain the elongate element 105. The method 200 provides for the advantageous benefits as discussed above in relation to the annuloplasty device 100 and Figs. 1 - 10. The method 200 allows for a facilitated anchoring of the annuloplasty device 100 at the heart valve, without the need to apply sutures, clips or other external fastening devices. A precise and improved control of the position of the retention units 104, 104', is provided.

Fig. 11b is a further flow chart of a method 200 of repairing a defective heart valve. The method 200 may comprise restraining 2011 the retention units 104, 104', to assume the retracted position by the elongate element 105 in the first state. Moving the elongate element 105 between the first state and a second state may comprise moving 2021 the elongate element 105 along a longitudinal direction 111 of the interior channel 106 to release 2022 the retention units 104, 104', to assume the expanded position.

Fig. 11b is a further flow chart of a method 200 of repairing a defective heart valve. Moving the elongate element 105 between a first state and a second state may comprise expanding 2021' the elongate element 105 in a radial direction 112 perpendicular to a longitudinal direction 111 of the interior channel 106 to push 2022' the retention units 104, 104', from the retracted position to the expanded position.

The method 200 may comprise gradually expanding 2021" the elongate element 105 to position 2022" the retention units 104, 104', at intermediate positions pₙ between the retracted position pᵣ to the expanded position pₑ.

## Claims

1. An annuloplasty device (100) comprising
first (101) and second (102) support rings having a coiled configuration in which the first and second support rings are arranged as a coil around a central axis (103), wherein the first and second support rings are configured to be arranged on opposite sides of native heart valve leaflets (301) of a heart valve,
retention units (104, 104'),
an elongate element (105) to apply a force onto the retention units and being movable between a first state and a second state, **characterized in that**
at least part of the first and second support rings comprises an interior channel (106) configured to contain the elongate element,
wherein, in the first state of the elongate element, the retention units have a retracted position, in which the retention units are arranged radially within an outer surface (107) of the first and/or second support rings,
wherein, in the second state of the elongate element, the retention units have an expanded position, in which the retention units protrude from the outer surface of the first and/or second support rings,
wherein the elongate element is movable between the first and second state by being movable along a longitudinal direction (111) of the interior channel.

2. Annuloplasty device according to claim 1, wherein the retention units are integrated with the first and/or second support rings.

3. Annuloplasty device according to claim 1 or 2, wherein the first and/or second support rings are formed from a material with circumferential walls (108) enclosing said interior channel, wherein the retention units are formed from the material of the circumferential walls.

4. Annuloplasty device according to claim 3, wherein the circumferential walls have a tubular shape enclosing said interior channel.

5. Annuloplasty device according to claim 2, wherein the circumferential walls comprise a plurality of sides (109, 109') forming a non-tubular shape enclosing said interior channel.

6. Annuloplasty device according to claim 5, wherein the non-tubular shape is essentially rectangular.

7. Annuloplasty device according to any of claims 1 - 6, comprising a core (110) extending along at least part of the first and/or second support rings.

8. Annuloplasty device according to claim 3 and 7, wherein the circumferential walls enclose the core.

9. Annuloplasty device according to claim 1, wherein the retention units are restrained by the elongate element to assume the retracted position in the first state, and, upon moving the elongate element along the longitudinal direction, the retention units are released to assume the expanded position.

10. Annuloplasty device according to any of claims 1-9, wherein the elongate element is movable between the first and second state by being expandable in a radial direction (112) perpendicular to a longitudinal direction (111) of the interior channel.

11. Annuloplasty device according to claim 10, wherein, upon expanding the elongate element in the radial direction, the retention units are pushed by the elongate element to transfer from the retracted position to the expanded position.

12. Annuloplasty device according to claim 10 or 11, wherein elongate element is gradually expandable to position the retention units at intermediate positions (pₙ) between the retracted position (pᵣ) to the expanded position (pₑ).

13. Annuloplasty device according to any of claims 1 - 12, wherein the retention units comprise a shape-memory material, wherein activation of the shape-memory material causes the retention units to transfer from the retracted state to the expanded state

14. Annuloplasty device according to any of claims 1 - 13, wherein the first support ring is adapted to be arranged on an atrial side of said heart valve, and the second support ring is adapted to be arranged on a ventricular side of the heart valve,
wherein the first support ring comprises a first posterior bow (113) and the second support ring comprises a second posterior bow (113'), wherein the first and second posterior bows are adapted to conform to a posterior aspect of said heart valve, and wherein the first and second posterior bows are separated by an intermediate anterior portion (114),
wherein the anterior portion comprises a smooth surface.

15. Annuloplasty device according to any of claims 1 - 14, wherein the first support ring comprises first retention units (104),
wherein the second support ring comprises second retention units (104'),
wherein the first and second retention units extend from respective first and second support rings to produce a retention force, in use, at both of said opposite sides.

## Patentansprüche

1. Annuloplastievorrichtung (100), umfassend
einen ersten (101) und einen zweiten (102) Stützring mit einer gewundenen Auslegung, in der der erste und der zweite Stützring als eine Spule um eine Mittelachse (103) angeordnet sind, wobei der erste und der zweite Stützring dazu ausgelegt sind, auf gegenüberliegenden Seiten der nativen Herzklappensegel (301) einer Herzklappe angeordnet zu werden,
Rückhalteeinheiten (104, 104'),
ein längliches Element (105) zum Aufbringen einer Kraft auf die Rückhalteeinheiten, das zwischen einem ersten Zustand und einem zweiten Zustand beweglich ist, **dadurch gekennzeichnet, dass** mindestens ein Teil des ersten und des zweiten Stützrings einen Innenkanal (106) umfasst, der dazu ausgelegt ist, das längliche Element zu enthalten,
wobei, in dem ersten Zustand des länglichen Elements, die Rückhalteeinheiten eine eingefahrene Position haben, in der die Rückhalteeinheiten radial im Innern einer Außenfläche (107) des ersten und/oder des zweiten Stützrings angeordnet sind,
wobei, in dem zweiten Zustand des länglichen Elements, die Rückhalteeinheiten eine ausgefahrene Position haben, in der die Rückhalteeinheiten von der Außenfläche des ersten und/oder des zweiten Stützrings vorspringen,
wobei das längliche Element zwischen dem ersten und dem zweiten Zustand beweglich ist, indem es entlang einer Längsrichtung (111) des Innenkanals beweglich ist.

2. Annuloplastievorrichtung nach Anspruch 1, wobei die Rückhalteeinheiten in dem ersten und/oder dem zweiten Stützring integriert sind.

3. Annuloplastievorrichtung nach Anspruch 1 oder 2, wobei der erste und/oder der zweite Stützring aus einem Material mit Umfangswänden (108) gebildet sind, die den Innenkanal umschließen, wobei die Rückhalteeinheiten aus dem Material der Umfangswände gebildet sind.

4. Annuloplastievorrichtung nach Anspruch 3, wobei die Umfangswände eine röhrenförmige Form haben, die den Innenkanal umschließt.

5. Annuloplastievorrichtung nach Anspruch 2, wobei die Umfangswände eine Vielzahl von Seiten (109, 109') umfassen, die eine nicht röhrenförmige Form bilden, die den Innenkanal umschließt.

6. Annuloplastievorrichtung nach Anspruch 5, wobei die nicht röhrenförmige Form im Wesentlichen rechtwinklig ist.

7. Annuloplastievorrichtung nach einem der Ansprüche 1-6, umfassend einen Kern (110), der sich entlang mindestens eines Teils des ersten und/oder des zweiten Stützrings erstreckt.

8. Annuloplastievorrichtung nach Anspruch 3 und 7, wobei die Umfangswände den Kern umschließen.

9. Annuloplastievorrichtung nach Anspruch 1, wobei die Rückhalteeinheiten von dem länglichen Element zurückgehalten werden, um die eingefahrene Position in dem ersten Zustand anzunehmen, und, beim Bewegen des länglichen Elements entlang der Längsrichtung, die Rückhalteeinheiten freigegeben werden, um die ausgefahrene Position anzunehmen.

10. Annuloplastievorrichtung nach einem der Ansprüche 1-9, wobei das längliche Element zwischen dem ersten und dem zweiten Zustand beweglich ist, indem es in eine radiale Richtung (112) senkrecht zu einer Längsrichtung (111) des Innenkanals ausfahrbar ist.

11. Annuloplastievorrichtung nach Anspruch 10, wobei, beim Ausfahren des länglichen Elements in die radiale Richtung, die Rückhalteeinheiten von dem länglichen Element gedrückt werden, um aus der eingefahrenen Position in die ausgefahrene Position zu wechseln.

12. Annuloplastievorrichtung nach Anspruch 10 oder 11, wobei das längliche Element schrittweise ausfahrbar ist, um die Rückhalteeinheiten in intermediären Positionen (pₙ) zwischen der eingefahrenen Position (pᵣ) und der ausgefahrenen Position (pₑ) zu positionieren.

13. Annuloplastievorrichtung nach einem der Ansprüche 1-12, wobei die Rückhalteeinheiten ein Formgedächtnismaterial umfassen, wobei die Aktivierung des Formgedächtnismaterials bewirkt, dass die Rückhalteeinheiten aus dem eingefahrenen Zustand in den ausgefahrenen Zustand wechseln.

14. Annuloplastievorrichtung nach einem der Ansprüche 1-13, wobei der erste Stützring dazu angepasst ist, auf einer atrialen Seite der Herzklappe angeordnet zu sein, und der zweite Stützring dazu angepasst ist, auf einer ventrikulären Seite der Herzklappe angeordnet zu sein,
wobei der erste Stützring einen ersten hinteren Bogen (113) umfasst und der zweite Stützring einen zweiten hinteren Bogen (113') umfasst, wobei der erste und der zweite hintere Bogen dazu angepasst sind, einem hinteren Aspekt der Herzklappe zu entsprechen, und wobei der erste und der zweite hintere Bogen durch einem intermediären vorderen Abschnitt (114) getrennt sind,
wobei der vordere Abschnitt eine glatte Oberfläche umfasst.

15. Annuloplastievorrichtung nach einem der Ansprüche 1-14, wobei der erste Stützring erste Rückhalteeinheiten (104) umfasst,
wobei der zweite Stützring zweite Rückhalteeinheiten (104') umfasst,
wobei sich die ersten und die zweiten Rückhalteeinheiten vom jeweiligen ersten und zweiten Stützring erstrecken, um, bei Verwendung eine Rückhaltekraft auf beiden gegenüberliegenden Seiten zu erzeugen.

## Revendications

1. Dispositif pour annuloplastie (100) comprenant
des premier (101) et second (102) anneaux de support ayant une configuration enroulée dans laquelle les premier et second anneaux de support sont agencés sous la forme d'un enroulement autour d'un axe central (103), dans lequel les premier et second anneaux de support sont configurés pour être agencés sur des côtés opposés de feuillets de valvule cardiaque native (201) d'une valvule cardiaque,
des unités de retenue (104, 104'),
un élément allongé (105) destiné à appliquer une force sur les unités de retenue et étant mobile entre un premier état et un second état, **caractérisé en ce que**
au moins une partie des premier et second anneaux de support comprend un canal intérieur (106) configuré pour contenir l'élément allongé,
dans lequel, dans le premier état de l'élément allongé, les unités de retenue ont une position rétractée, dans laquelle les unités de retenue sont agencées radialement à l'intérieur d'une surface externe (107) des premier et/ou second anneaux de support,
dans lequel, dans le second état de l'élément allongé, les unités de retenue ont une position étendue, dans laquelle les unités de retenue font saillie depuis la surface externe des premier et/ou second anneaux de support,
dans lequel l'élément allongé est mobile entre un premier état et un second état en étant mobile le long d'une direction longitudinale (111) du canal intérieur.

2. Dispositif pour annuloplastie selon la revendication 1, dans lequel les unités de retenue sont intégrées aux premier et/ou second anneaux de support.

3. Dispositif pour annuloplastie selon la revendication 1 ou 2, dans lequel les premier et/ou second anneaux de support sont formés à partir d'un matériau avec des parois circonférentielles (108) entourant ledit canal intérieur, dans lequel les unités de retenue sont formées à partir du matériau des parois circonférentielles.

4. Dispositif pour annuloplastie selon la revendication 3, dans lequel les parois circonférentielles ont une forme tubulaire entourant ledit canal intérieur.

5. Dispositif pour annuloplastie selon la revendication 2, dans lequel les parois circonférentielles comprennent une pluralité de côtés (109, 109') formant une forme non tubulaire entourant ledit canal intérieur.

6. Dispositif pour annuloplastie selon la revendication 5, dans lequel la forme non tubulaire est essentiellement rectangulaire.

7. Dispositif pour annuloplastie selon l'une quelconque des revendications 1 à 6, comprenant une partie centrale (110) s'étendant le long d'au moins une partie des premier et/ou second anneaux de support.

8. Dispositif pour annuloplastie selon les revendications 3 et 7, dans lequel les parois circonférentielles entourent la partie centrale.

9. Dispositif pour annuloplastie selon la revendication 1, dans lequel les unités de retenue sont limitées par l'élément allongé pour prendre la position rétractée dans le premier état, et, lors du déplacement de l'élément allongé le long de la direction longitudinale, les unités de retenue sont libérées pour prendre la position étendue.

10. Dispositif pour annuloplastie selon l'une quelconque des revendications 1 à 9, dans lequel l'élément allongé est mobile entre les premier et second états en pouvant être étendu dans une direction radiale (112) perpendiculaire à une direction longitudinale (111) du canal intérieur.

11. Dispositif pour annuloplastie selon la revendication 10, dans lequel, lors de l'extension de l'élément allongé dans la direction radiale, les unités de retenue sont poussées par l'élément allongé pour passer de la position rétractée à la position étendue.

12. Dispositif pour annuloplastie selon la revendication 10 ou 11, dans lequel l'élément allongé peut être progressivement étendu pour positionner les unités de retenue au niveau de positions intermédiaires (pₙ) entre la position rétractée (pᵣ) et la position étendue (pₑ) .

13. Dispositif pour annuloplastie selon l'une quelconque des revendications 1 à 12, dans lequel les unités de retenue comprennent un matériau à mémoire de forme, dans lequel l'activation du matériau à mémoire de forme amène les unités de retenue à passer de l'état rétracté à l'état étendu.

14. Dispositif pour annuloplastie selon l'une quelconque des revendications 1 à 13, dans lequel le premier anneau de support est conçu pour être agencé sur un côté auriculaire de ladite valvule cardiaque, et le second anneau de support est conçu pour être agencé sur un côté ventriculaire de la valvule cardiaque,
dans lequel le premier anneau de support comprend un premier arc postérieur (113) et le second anneau de support comprend un second arc postérieur (113'), dans lequel les premier et second arcs postérieurs sont conçus pour épouser la forme d'un aspect postérieur de ladite valvule cardiaque, et dans lequel les premier et second arcs postérieurs sont séparés par une partie antérieure intermédiaire (114),
dans lequel la partie antérieure comprend une surface lisse.

15. Dispositif pour annuloplastie selon l'une quelconque des revendications 1 à 14, dans lequel le premier anneau de support comprend des premières unités de retenue (104),
dans lequel le second anneau de support comprend des secondes unités de retenue (104'),
dans lequel les première et seconde unités de retenue s'étendent à partir de premier et second anneaux de support respectifs pour produire une force de retenue, lors de l'utilisation, au niveau desdits deux côtés opposés.
